# EUROPÄISCHE PATENTSCHRIFT

(11) **EP 2 346 543 B1**
(45) Veröffentlichungstag und Bekanntmachung des Hinweises auf die Patenterteilung: **23.07.2014**
(21) Anmeldenummer: 09744076.2
(22) Anmeldetag: 27.10.2009
(51) Int. Cl.: A61L 27/20, A61L 27/56, A61L 27/58, B22F 7/06

(54) **VERFAHREN ZUR HERSTELLUNG EINES HALBZEUGS**
METHOD FOR PRODUCING A SEMI-FINISHED PART
PROCÉDÉ POUR PRODUIRE UN DEMI-PRODUIT

(30) Priorität: 06.11.2008 DE 102008056263
(43) Veröffentlichungstag der Anmeldung: 27.07.2011
(73) Patentinhaber: DODUCO GmbH, 75181 Pforzheim (DE)
(72) Erfinder: HEINZEL, Helmut, 75233 Tiefenbronn (DE); MOOG, Dirk, 75196 Remchingen (DE); WITULSKI, Norbert, 75180 Pforzheim (DE); DASLER, Stefan, 75175 Pforzheim (DE); KRAUS, Andreas, 75417 Mühlacker (DE); WENZ, Johann, 75179 Pforzheim (DE); MAHLE-MÖSSNER, Evelin, 75181 Pforzheim (DE); BEHRENS, Volker, 75015 Bretten (DE)
(74) Vertreter: Twelmeier Mommer & Partner
(86) Internationale Anmeldenummer: PCT/EP2009/007661
(87) Internationale Veröffentlichungsnummer: WO 2010/051922

(56) Entgegenhaltungen:
- EP-A1- 0 299 099
- DE-A1- 3 212 005
- GB-A- 991 433
- US-A- 3 226 517
- US-A- 3 335 001

## Beschreibung

Die Erfindung betrifft ein Verfahren zur Herstellung eines Halbzeugs für elektrische Kontakte mit einer für die elektrische Kontaktgabe bestimmten Oberseite aus einem Verbundwerkstoff auf Silberbasis, in welchem eines oder mehrere Metalloxide oder Kohlenstoff eingelagert sind, und einer Trägerschicht aus gut lötbaren oder schweißbaren Metall, auf welcher der Verbundwerkstoff liegt.

Verbundwerkstoffe aus Silberbasis mit eingelagerten Metalloxid- oder Kohlenstoffpartikeln lassen sich nicht oder nur sehr schlecht verschweißen und verlöten. Bei der Herstellung von Halbzeugen für elektrische Kontakte wird deshalb eine Unterseite des Kontaktwerkstoffs mit einer Trägerschicht aus einem gut lötbaren oder schweißbaren Metall versehen. Während sich Trägerschichten aus Silber durch Aufplattieren eines Silberbandes verhältnismäßig einfach auf Kontaktwerkstoffe aufbringen lassen, bereitet das Aufbringen einer schweißbaren Trägerschicht aus wesentlich kostengünstigerem Kupfer oder anderen unedlen Metallen erhebliche Probleme. Im Gegengünstigerem Kupfer oder anderen unedlen Metallen erhebliche Probleme. Im Gegensatz zu Silber oxidiert Kupfer an Luft nämlich recht schnell, so dass Kupferbänder wegen einer Oxidschicht selbst unter Einwirkung von Druck und erhöhter Temperaturen nicht oder nur sehr schlecht an Kontaktwerkstoffen haften.

Aus der EP 0 299 099 B1 ist es bekannt, Kontaktplättchen aus einem Verbundwerkstoff auf Silberbasis, in welchem Metalloxidpartikel eingelagert sind, mit einer lötfähigen oder schweißfähigen Trägerschicht zu versehen, indem das Kontaktplättchen bei erhöhten Temperaturen unter einer Inertgasatmosphäre oder einer reduzierenden Atmosphäre mit einer Metallfolie oder einer Schicht aus Metallpulver verpresst wird. Die Herstellung eines Halbzeugs durch Einzelpresstechnik ist auch aus der DE 32 12 005 C2 bekannt, welche die Herstellung eines Zweischichtenformkörpers durch Verpressen einer unteren Schicht aus Kupferpulver und einer oberen Schicht aus einem Verbundpulver mit Silber und Metalloxid sowie anschließendes Sintern in einer inerten Atmosphäre offenbart.

Aufgabe der Erfindung ist es, einen Weg aufzuzeigen, wie kostengünstig ein strangförmiges Halbzeug für elektrische Kontakte hergestellt werden kann, das eine für die elektrische Kontaktgabe bestimmte Oberseite aus einem Verbundwerkstoff auf Silberbasis und eine Unterseite aus gut lötbaren oder schweißbaren unedlen Metall hat.

Diese Aufgabe wird durch ein Verfahren mit den im Anspruch 1 angegebenen Merkmalen gelöst. Vorteilhafte Weiterbildungen der Erfindung sind Gegenstand der Unteransprüche.

Bei einem erfindungsgemäßen Verfahren wird zunächst pulvermetallurgisch ein Block aus einem Verbundwerkstoff auf Silberbasis hergestellt, beispielsweise indem Silberpulver mit Metalloxidpulver vermischt, verpresst und anschließend gesintert wird. Möglich ist es beispielsweise auch, Silberpulver mit einem unedlen Metallpulver zu vermischen, zu verpressen und anschließend in einer oxidierenden Atmosphäre zu sintern, so dass durch Oxidation der unedlen Metallpartikel Metalloxidpartikel entstehen.

In einem zweiten Schritt wird der pulvermetallurgisch hergestellte Block mit einem Pulver aus einem gut lötbarem oder schweißbarem unedlem Metall, das bei dem fertigen Halbzeug die Trägerschicht bilden soll, ummantelt und anschließend isostatisch gepresst, bevorzugt bei Drücken von 500 bar bis 2500 bar. Als unedles Metall zur Ausbildung der Trägerschicht können beispielsweise Nickel, Eisen oder Kupfer sowie Legierungen dieser Metalle verwendet werden, wobei Kupfer und Kupferbasislegierungen, insbesondere Messing, besonders bevorzugt sind.

Der bevorzugt isostatisch gepresste Block wird in einem weiteren Herstellungsschritt unter nicht-oxidierenden Bedingungen gesintert. Der gepresste Block kann also beispielsweise in reduzierender Atmosphäre, inerter Atmosphäre oder in Vakuum gesintert werden. Das Sintern wird unter Vermeidung der Bildung eines flüssigen Eutektikums aus dem Silber des Verbundwerkstoffs und aus dem unedlen Metall, mit welchem der Block aus dem Verbundwerkstoff auf Silberbasis ummantelt ist, durchgeführt.

Die Bildung eines flüssigen Eutektikums kann am einfachsten dadurch vermieden werden, dass das Sintern bei einer Temperatur durchgeführt wird, die geringer als die eutektische Bildungstemperatur ist; also beispielsweise bei Verwendung von Kupfer als unedlem Metall bei einer Temperatur von weniger als 779°C, der Bildungstemperatur des Silber-Kupfer Eutektikums. Die Bildung eines flüssigen Eutektikums kann aber auch dadurch vermieden werden, dass zwischen dem Block aus Verbundwerkstoff und dem aus unedlem Metallpulver gebildeten Mantel eine als Diffusionsbarriere wirkende Zwischenschicht vorgesehen wird. Eine solche Zwischenschicht kann insbesondere dadurch gebildet werden, dass der Block aus dem Verbundwerkstoff mit einer Folie aus einem die Diffusionsbarriere bildenden Werkstoff umwickelt wird. Das unedle Metallpulver kann nach dem Umwickeln des Verbundwerkstoffblocks um den umwickelten Block herum angeordnet und durch isostatisches Pressen mit diesem Verbunden werden. Als Diffusionsbarriere sind insbesondere Zwischenschichten aus Eisen, Nickel oder Kobalt und Legierungen dieser Metalle geeignet, wobei Nickel und Nickellegierungen besonders bevorzugt sind. Die als Diffusionsbarriere wirkende Zwischenschicht wird bevorzugt so bemessen, dass sie im fertigen Halbzeug mindestens 3 µm, insbesondere minedestens 5 µm und vorzugsweise 10 µm bis 50 µm dick ist.

Besonders bevorzugt wird zunächst in einer reduzierenden Atmosphäre, insbesondere unter Wasserstoff, bei einer Temperatur von weniger als 700°C gesintert und anschließend unter einer inerten Atmosphäre, insbesondere unter Stickstoff, oder Vakuum bei einer höheren Temperatur, vorzugsweise bei mindestens 750°C, gesintert. Auf diese Weise können bei einem ersten Sinterschritt unter reduzierender Atmosphäre eventuell vorhandene Oxidschichten der Körner des unedlen Metallpulvers, beispielsweise Kupfer, reduziert werden, ohne dass Metalloxidpartikel, beispielsweise Zinn- oder Zinkoxidpartikel, des Verbundwerkstoffs reduziert werden. Bei einem zweiten Sinterschritt unter inerter Atmosphäre oder unter Vakuum kann die Sintertemperatur erhöht werden, damit sich die Körner des Metallpulver fest miteinander und dem Verbundwerkstoffblock verbinden.

Nach dem Sintern wird der Block durch Strangpressen umgeformt, vorzugsweise warmumgeformt. Danach wird mindestens ein Teilstrang mit einer Oberseite aus Verbundwerkstoff und einer gut löt- oder schweißbaren Unterseite aus unedlem Metall erzeugt. Bevorzugt werden zwei derartige Teilstränge erzeugt, indem der durch Strangpressen gebildete Strang in seiner Längsrichtung geteilt wird. Dabei ist es selbstverständlich möglich, senkrecht zu der Teilungsebene weitere Längsteilungen vorzunehmen. Die Angabe, dass bevorzugt zwei Teilstränge erzeugt werden, ist deshalb im Sinne von mindestens zwei zu verstehen. Möglich ist es aber auch, nur einen Teilstrang zu erzeugen, indem auf einer Seite des durch Strangpressen gebildeten Strangs das unedle Metall abgetragen wird, beispielsweise durch Fräsen, und so eine Oberfläche aus Verbundwerkstoff freigelegt wird

Nach dem Sintern haftet das unedle Metall in der Regel bereits ausreichend stark an dem Block, so dass dieser formgebend bearbeitet werden kann, damit er passgenau in ein Strangpresswerkzeug eingesetzt werden kann. Beispielsweise kann durch Pressen und anschließendes Sintern ein annährend zylindrischer Block hergestellt werden, dessen Mantelfläche vor dem Strangpressen abgedreht wird, um eine Anpassung an die Maße eines Strangpresswerkzeugs zu erreichen. Bevorzugt wird der Block durch das Strangpressen von einer zylindrischen Form in eine Form mit rechteckigem Querschnitt umgeformt.

Das Strangpressen wird bevorzugt bei Temperaturen von mindestens 600°C, insbesondere zwischen 700°C und 950°C, durchgeführt. Diese Maßnahme hat den Vorteil, dass durch das Strangpressen eine vorteilhaft hohe Verdichtung erreicht wird. Vorteilhaft lässt sich insbesondere erreichen, dass der Strang eine relative Dichte von 99,9 % der theoretisch möglichen Dichte hat.

Durch Teilen des durch Strangpressen gebildeten Strangs in seiner Längsrichtung entsteht ein Halbzeug mit einer Schicht aus dem Verbundwerkstoff auf Silberbasis, welche die zur Kontaktgabe bestimmte Oberseite des Halbzeugs bildet, und einer Trägerschicht aus gut lötbarem oder schweißbarem, unedlem Metall.

Bevorzugt werden die beiden Flanken des Strangs, welche sich von der kontaktgebenden Oberseite bis zur gut löt- und schweißbaren Unterseite des Strangs erstrecken, getrimmt, insbesondere durch Schneiden oder Fräsen. Auf diese Weise kann sichergestellt werden, dass bei der weiteren Verarbeitung des Halbzeugs oder beim späteren Gebrauch eines mit dem Halbzeug hergestellten elektrischen Kontakts kein Material der Trägerschicht auf die Kontaktfläche gelangt und deren Funktion beeinträchtigt. Das Trimmen der Flanken des durch Strangpressen gebildeten Strangs kann wahlweise vor oder nach der Längsteilung des Strangs durchgeführt werden.

Bevorzugt wird der durch Strangpressen erzeugte Strang in seiner Dicke durch Walzen, insbesondere durch Kaltwalzen, reduziert. Auf diese Weise lässt sich besonders günstig ein bandförmiges Halbzeug herstellen. Bevorzugt ist dabei insbesondere, dass das Walzen nach dem Längsteilen des Strangs erfolgt, also der oder die Teilstränge gewalzt werden. Bevorzugt ist ferner, dass der Strang beim Walzen in seiner Dicke um höchstens 50% seiner ursprünglichen Dicke reduziert wird, um zu vermeiden dass mechanische Eigenschaften des Halbzeugs nachteilig beeinträchtigt werden. Insbesondere besteht bei einer Reduktion der Dicke um mehr als 50% die Gefahr, dass das Material zu hart wird. Besonders bevorzugt wird der Strang beim Walzen in seiner Dicke um 30 bis 50% seiner ursprünglichen Dicke reduziert.

Eine vorteilhafte Weiterbildung der Erfindung sieht vor, dass zum Ummanteln des Verbundwerkstoffblocks oxidationsgeschütztes Metallpulver verwendet wird, also die Partikel des Pulvers aus dem unedlen Metall, mit dem der Block aus dem Verbundwerkstoff ummantelt wird, durch einen organischen Überzug vor Oxidation geschützt sind. Auf diese Weise kann der Bildung von störenden Oxidschichten, die den Sinterprozess erschweren, entgegengewirkt werden. Bevorzugt wird der organische Überzeug so gewählt, dass er sich beim Sintern rückstandslos verflüchtigt, beispielsweise in dem er verdampft.

Bevorzugt wird bei dem Verfahren ein Verbundwerkstoff verwendet, der ein Silber-Metalloxid-Verbundwerkstoff ist. Als Metalloxide können insbesondere Zinnoxid und/oder Zinkoxid und/oder Indiumoxid und/oder Kadmiumoxid verwendet werden. Möglich sind beispielsweise auch die Verwendung von Bismutoxid oder Wolframoxid. Dabei ist es möglich, dass der erfindungsgemäß verwendete Verbundwerkstoff mehrere Metalloxide enthält. Ebenso gut ist es möglich, dass der Verbundwerkstoff nur ein einziges Metalloxid enthält. Bevorzugt besteht die Metalloxidkomponente des Verbundwerkstoffs überwiegend aus Zinnoxid. Als Alternative oder zusätzlich zu Metalloxiden kann der verwendete Kontaktwerkstoff auf Silberbasis auch Kohlenstoff enthalten, beispielsweise in Form von Grafit.

Bevorzugt wird der mit unedlem Metallpulver ummantelte Verbundwerkstoffblock kaltisostatisch gepresst. Das kaltisostatische Pressen kann problemlos bei Raumtemperatur durchgeführt werden. Prinzipiell kann das kaltisostatische Pressen auch bei erhöhten Temperaturen durchgeführt werden, bevorzugt ist aber jedenfalls, dass das kaltisostatische Pressen bei einer Temperatur durchgeführt wird, bei welcher das unedle Metall in Anwesenheit von Luftsauerstoff allenfalls unwesentlich oxidiert wird.

### Ausführungsbeispiele:

1. Durch Mischen von Silberpulver und Zinnoxidpulver, kaltisostatisches Pressen und anschließendes Sintern wird ein zylindrischer Block aus einem Kontaktwerkstoff auf Silberbasis hergestellt. Dieser Block kann beispielweise zu 8 bis 14 Gewichtsprozent aus Metalloxid und im übrigen aus Silber bestehen.
   Der Verbundwerkstoffblock wird mit einer Folie aus Nickel oder einer Nickeleisenlegierung umwickelt. Der umwickelte Block wird anschließend mit oxidationsgeschütztem Kupfer- oder Messingpulver ummantelt und dann kaltisostatisch verpresst.
   Der isostatisch gepresste Block wird in einer reduzierenden Atmosphäre, beispielsweise unter Wasserstoff, bei einer Temperatur von 700°C, beispielsweise für 30 Minten bis 1 Stunde, und anschließend unter Stickstoff bei 800°C bis 900°C gesintert, beispielsweise für 2 bis 5 Stunden. Der gesinterte Block wird anschließend abgedreht, damit er maßgenau in eine Strangpresse eingesetzt werden kann. Der Block wird dann durch Strangpressen bei einer Temperatur von 750° bis 775° C von seiner zylindrischen Form in eine Form mit rechteckigem Querschnitt umgeformt.
   Die Flanken des so erzeugten Strangs werden abgeschnitten und der Strang anschließend in Längsrichtung geteilt. Die auf diese Weise gebildeten Teilstränge werden anschließend kaltgewalzt und dabei in ihrer Dicke um 30 bis 50%, beispielsweise um 45%, reduziert. Das aus diese Weise hergestellte bandförmige Halbzeug hat eine Trägerschicht, deren Dicke etwa 10% bis 20% der Dicker der Verbundwerkstoffschicht ausmacht, und kann zur Herstellung elektrischer Kontaktstücke verwendet werden, indem von dem Halbzeug Abschnitte abgeschnitten und gemäß den Anforderungen einer spezifischen Anwendung umgeformt werden.
2. Durch Mischen von Silberpulver und Graphitpulver, kaltisostatisches Pressen, anschließendes Sintern wird ein zylindrischer Block aus einem Kontaktwerkstoff auf Silberbasis hergestellt. Dieser Block kann beispielweise zu 2 bis 5 Gewichtsprozent aus Kohlenstoff und im Übrigen aus Silber bestehen. Der Verbundwerkstoffblock wird mit Pulver aus Kupfer oder einer Kupferbasislegierung ummantelt und einige Stunden unter einer Wasserstoffatmosphäre bei 750° bis 775° gesintert. Die weitere Verarbeitung des gesinterten Blocks kann wie bei dem vorstehend beschriebenen Ausführungsbeispiel erfolgen.

## Patentansprüche

1. Verfahren zur Herstellung eines strangförmigen, insbesondere bandförmigen, Halbzeugs für elektrische Kontakte, wobei das Halbzeug eine für die elektrische Kontaktgabe bestimmte Oberseite aus einem Verbundwerkstoff auf Silberbasis hat, in welchem eines oder mehrere Metalloxide oder Kohlenstoff eingelagert sind, und einer den Verbundwerkstoff tragenden Trägerschicht aus gut lötbarem oder schweißbarem, unedlem Metall hat, mit den folgenden Schritten:
- Pulvermetallurgisches Herstellen eines Blocks aus dem Verbundwerkstoff auf Silberbasis,
- Ummanteln des Blocks aus dem Verbundwerkstoff mit einem Pulver aus dem gut lötbaren oder schweißbaren unedlen Metall,
- Pressen des mit dem Metallpulver ummantelten Blocks zum Verdichten des Metallpulvers,
- Sintern des gepressten Blocks in reduzierender Atmosphäre oder in inerter Atmosphäre oder im Vakuum unter Vermeidung der Bildung eines flüssigen Eutektikums aus dem Silber des Verbundwerkstoffs und aus dem unedlen Metall, mit welchem der Block aus dem Verbundwerkstoff auf Silberbasis ummantelt ist,
- Umformen des gesinterten Blocks durch Strangpressen,
- Erzeugen eines Teilstrangs mit einer Oberseite aus Verbundwerkstoff und einer Unterseite aus unedlem Metall.

2. Verfahren nach Anspruch 1, **dadurch gekennzeichnet, dass** zwei Teilstränge erzeugt werden, indem der durch Strangpressen gebildete Strang in seiner Längsrichtung geteilt wird.

3. Verfahren nach einem der Ansprüche, **dadurch gekennzeichnet, dass** der Verbundwerkstoff ein Silber-Metalloxid Verbundwerkstoff ist.

4. Verfahren nach Anspruch 3, **dadurch gekennzeichnet, dass** das Sintern in einer reduzierenden Atmosphäre bei einer Temperatur von weniger als 700°C durchgeführt wird.

5. Verfahren nach Anspruch 4, **dadurch gekennzeichnet, dass** zunächst in einer reduzierenden Atmosphäre bei einer Temperatur von weniger als 700°C gesintert und anschließend in einer inerten Atmosphäre oder unter Vakuum bei einer höheren Temperatur gesintert wird.

6. Verfahren nach einem der vorstehenden Ansprüche, **dadurch gekennzeichnet, dass** zum Ummanteln des Blocks aus dem Verbundwerkstoff auf Silberbasis ein Pulver aus Nickel, Eisen, Kupfer oder einer Kupferbasislegierung verwendet wird, wobei Kupfer besonders bevorzug ist.

7. Verfahren nach Anspruch 6, **dadurch gekennzeichnet, dass** als Kupferbasislegierung eine Legierung von Kupfer mit Silber oder von Kupfer mit Zinn oder von Kupfer mit Nickel verwendet wird.

8. Verfahren nach einem der vorstehenden Ansprüche, **dadurch gekennzeichnet, dass** die Partikel des Metallpulvers durch einen organischen Überzug vor Oxidation geschützt sind.

9. Verfahren nach einem der vorstehenden Ansprüche, **dadurch gekennzeichnet, dass** der ummantelte Block isostatisch, vorzugsweise kaltisostatisch, gepresst wird.

10. Verfahren nach einem der vorstehenden Ansprüche, **dadurch gekennzeichnet, dass** das Sintern bei einer Temperatur durchgeführt wird, bei welcher sich kein flüssiges Eutektikum aus Silber und dem unedlen Metall bilden kann.

11. Verfahren nach einem der vorstehenden Ansprüche, **dadurch gekennzeichnet, dass** die Dicke des durch Strangpressen erzeugten Strangs oder eines Teilstrangs durch Walzen, insbesondere durch Kaltwalzen, reduziert wird.

12. Verfahren nach einem der vorstehenden Ansprüche, **dadurch gekennzeichnet, dass** die beiden Flanken des Stranges, welche sich von der Kontakt gebenden Oberseite bis zur von der Trägerschicht gebildeten gut löt- und schweißbaren Unterseite des Stranges erstrecken, getrimmt werden, insbesondere durch Schneiden oder Fräsen.

13. Verfahren nach einem der vorstehenden Ansprüche, **dadurch gekennzeichnet, dass** der Block durch das Strangpressen von einer zylindrischen Form in eine Form mit rechteckigem Querschnitt umgeformt wird.

14. Verfahren nach einem der vorstehenden Ansprüche, **dadurch gekennzeichnet, dass** zwischen dem Verbundblock und dem aus unedlem Metallpulver gebildeten Mantel eine als Diffusionsbarriere wirkende Zwischenschicht vorgesehen wird.

## Claims

1. A method for producing a strand-shaped, particularly a strip-shaped, semifinished product for electrical contacts, wherein the semifinished product has an upper face comprising a silver-based composite material intended for the electrical contact-making, one or more metal oxides or carbon being embedded in the composite material, and a carrier layer comprising easy-to-solder or easy-to-weld base metal, which carries the composite material, comprising the following steps:
- producing a block from the silver-based composite material using a powder-metallurgical process;
- ensheathing the block comprising the composite material with a powder of the easy-to-solder or easy-to-weld base metal;
- pressing the block covered with the metal powder so as to compact the metal powder;
- sintering the pressed block in a reducing atmosphere or in an inert atmosphere or in a vacuum preventing the formation of a liquid eutectic from the silver of the composite material and from the base metal with which the block comprising the silver-based composite material is covered;
- working the sintered block by extrusion molding;
- generating a partial strand having an upper face comprising composite material and a lower face comprising base metal.

2. The method according to claim 1, **characterized in that** two partial strands are generated by dividing the strand formed by extrusion molding in the longitudinal direction thereof.

3. A method according to any one of the claims, **characterized in that** the composite material is a silver-metal oxide composite material.

4. The method according to claim 3, **characterized in that** sintering is carried out in a reducing atmosphere at a temperature of less than 700°C.

5. The method according to claim 4, **characterized in that** sintering takes place first in a reducing atmosphere at a temperature of less than 700°C, and subsequently sintering takes place in an inert atmosphere or under a vacuum at a higher temperature.

6. A method according to any one of the preceding claims, **characterized in that** a powder comprising nickel, iron, copper or a copper-base alloy is used for ensheathing the block comprising the silver-based composite material, with copper being particularly preferred.

7. The method according to claim 6, **characterized in that** an alloy of copper with silver, or of copper with tin, or of copper with nickel is used as the copper-base alloy.

8. A method according to any one of the preceding claims, **characterized in that** the particles of the metal powder are protected from oxidation by an organic coating.

9. A method according to any one of the preceding claims, **characterized in that** the ensheathed block is isostatically pressed, preferably cold isostatically pressed.

10. A method according to any one of the preceding claims, **characterized in that** the sintering is carried out at a temperature at which no liquid eutectic can form from silver and the base metal.

11. A method according to any one of the preceding claims, **characterized in that** the thickness of the strand generated by extrusion molding, or a partial strand, is reduced by rolling, and more particularly by cold rolling.

12. A method according to any one of the preceding claims, **characterized in that** the two flanks of the strand extending from the contact-making upper face to the easy-to-solder and easy-to-weld lower face of the strand forming the carrier layer are trimmed, in particular by cutting or milling.

13. A method according to any one of the preceding claims, **characterized in that** the block is formed by extrusion molding from a cylindrical shape into a shape having a rectangular cross-section.

14. A method according to any one of the preceding claims, **characterized in that** an intermediate layer acting as a diffusion barrier is provided between the composite material block and the cover formed of base metal powder.

## Revendications

1. Procédé pour la fabrication d'un produit semi-fini en forme de brin, en particulier en forme de bande, pour des contacts électriques, dans lequel le produit semi-fini possède une surface supérieure destinée à la mise en contact électrique, constituée d'un matériau composite à base d'argent, dans lequel sont stockés un ou plusieurs oxydes métalliques ou du carbone, et possède une couche de support portant le matériau composite, constituée d'un métal non noble facile à braser ou à souder, avec les étapes suivantes :
- fabrication, par métallurgie des poudres, d'un bloc de matériau composite à base d'argent,
- enrobage du bloc de matériau composite avec une poudre constituée du métal non noble approprié pour le soudage ou le brasage,
- pressage du bloc enrobé de poudre métallique, pour le compactage de la poudre métallique,
- frittage du bloc pressé dans une atmosphère réduite ou dans une atmosphère inerte ou sous vide, en évitant la formation d'une eutexie sur l'argent du matériau composite et sur le métal non noble, dans lequel le bloc en matériau composite à base d'argent est enrobé,
- déformation du bloc fritté par extrusion,
- fabrication d'un brin partiel avec un côté supérieur en matériau composite et un côté inférieur en métal non noble.

2. Procédé selon la revendication 1, **caractérisé en ce que** deux brins partiels sont produits en divisant le brin formé par extrusion dans son sens longitudinal.

3. Procédé selon l'une des revendications, **caractérisé en ce que** le matériau composite est un matériau composite d'argent-oxyde métallique.

4. Procédé selon la revendication 3, **caractérisé en ce que** le frittage est effectué dans une atmosphère réduite, à une température inférieure à 700°C.

5. Procédé selon la revendication 4, **caractérisé en ce que** le frittage a lieu tout d'abord dans une atmosphère réduite, à une température inférieure à 700°C, puis le frittage a lieu dans une atmosphère inerte ou sous vide, à une température plus élevée.

6. Procédé selon l'une des revendications précédentes, **caractérisé en ce qu'**une poudre à base de nickel, de fer, de cuivre ou d'alliage à base de cuivre est utilisée pour l'enrobage du bloc en matériau composite à base d'argent, le cuivre étant particulièrement préféré.

7. Procédé selon la revendication 6, **caractérisé en ce qu'**un alliage de cuivre et d'argent ou de cuivre et d'étain ou de cuivre et de nickel est utilisé comme alliage à base de cuivre.

8. Procédé selon l'une des revendications précédentes, **caractérisé en ce que** les particules de poudre métallique sont protégées contre l'oxydation par un revêtement organique.

9. Procédé selon l'une des revendications précédentes, **caractérisé en ce que** le bloc enrobé est pressé de façon isostatique, de préférence de façon isostatique à froid.

10. Procédé selon l'une des revendications précédentes, **caractérisé en ce que** le frittage est effectué à une température à laquelle aucune eutexie liquide ne peut se former sur l'argent et le métal non noble.

11. Procédé selon l'une des revendications précédentes, **caractérisé en ce que** l'épaisseur du brin fabriqué par extrusion ou d'un brin partiel est réduite par laminage, en particulier par laminage à froid.

12. Procédé selon l'une des revendications précédentes, **caractérisé en ce que** les deux flancs du brin s'étendant de la surface supérieure de mise en contact jusqu'au côté inférieur du brin facile à braser et à souder, formé par la couche de support, sont taillés, en particulier par découpage ou fraisage.

13. Procédé selon l'une des revendications précédentes, **caractérisé en ce que** le bloc est déformé par l'extrusion, d'une forme cylindrique en une forme à section transversale rectangulaire.

14. Procédé selon l'une des revendications précédentes, **caractérisé en ce qu'**une couche intermédiaire agissant comme barrière de diffusion est prévue entre le bloc composite et l'enveloppe constituée de poudre de métal non noble.
